# NEUE EUROPÄISCHE PATENTSCHRIFT

(11) **EP 1 410 721 B2**
(45) Veröffentlichungstag und Bekanntmachung des Hinweises auf die Entscheidung über den Einspruch: **08.08.2012**
(45) Hinweis auf die Patenterteilung: 24.05.2006
(21) Anmeldenummer: 03020046.3
(22) Anmeldetag: 04.09.2003
(51) Int. Cl.: A23L 1/236, A23L 1/29

(54) **Verfahren zur Herstellung einer Isomaltulose-haltigen Enteralnahrung**
Process for manufacturing an isomaltulose-containing enteral food
Procédé pour la préparation d'un aliment enteral comprenant d'isomaltulose

(30) Priorität: 17.10.2002 DE 10248515; 30.10.2002 DE 10251648; 12.03.2003 DE 10310648
(43) Veröffentlichungstag der Anmeldung: 21.04.2004
(73) Patentinhaber: Südzucker Aktiengesellschaft Mannheim/Ochsenfurt, 68165 Mannheim (DE)
(72) Erfinder: Kowalczyk, Jörg, Dr., 67304 Eisenberg/Steinborn (DE); Kozianowski, Gunhild, Dr., 67269 Grünstadt (DE); Kunz, Markwart, Prof. Dr., 67550 Worms (DE); Moser, Matthias, Dr., 67269 Grünstadt (DE)
(74) Vertreter: Schwahn, Hartmut

(56) Entgegenhaltungen:
- US-A- 4 948 616
- US-A1- 2001 005 524
- DATABASE BIOSIS [Online] BIOSCIENCES INFORMATION SERVICE, PHILADELPHIA, PA, US; 1985 KAWAI K ET AL: "CHANGES IN BLOOD GLUCOSE AND INSULIN AFTER AN ORAL PALATINOSE ADMINISTRATION IN NORMAL SUBJECTS" Database accession no. PREV198682055038 XP002263919 & ENDOCRINOLOGIA JAPONICA, Bd. 32, Nr. 6, 1985, Seiten 933-936, ISSN: 0013-7219
- PATENT ABSTRACTS OF JAPAN vol. 013, no. 487 (C-649), 6. November 1989 (1989-11-06) & JP 01 191646 A (MITSUI SEITO KK), 1. August 1989 (1989-08-01)
- DATABASE WPI Section Ch, Week 199837 Derwent Publications Ltd., London, GB; Class D13, AN 1998-430844 XP002263922 & JP 10 179036 A (FUJIYA KK), 7. Juli 1998 (1998-07-07)
- DATABASE FSTA [Online] INTERNATIONAL FOOD INFORMATION SERVICE (IFIS), FRANFURT/MAIN, DE; CRITTENDEN R G ET AL: "Production, properties and applications of food-grade oligosaccharides." Database accession no. 97-1-02-a0013 XP002263920 & TRENDS IN FOOD SCIENCE & TECHNOLOGY 1996 MELBOURNE LAB., CSIRO DIV. OF FOOD SCI. & TECH., PO BOX 20, HIGHETT, VIC. 3190, AUSTRALIA. FAX +61-9252-6555. E-MAIL ROSSC(A)MEL.DBCE.CSIRO.AU, Bd. 7, Nr. 11, Seiten 353-361,
- DATABASE FSTA [Online] INTERNATIONAL FOOD INFORMATION SERVICE (IFIS), FRANFURT/MAIN, DE; SINGH K ET AL: "Microbiological evaluation of raw, pasteurized and flavoured milk." Database accession no. 76-4-11-p2195 XP002263921 & JOURNAL OF FOOD SCIENCE AND TECHNOLOGY, INDIA 1975 INST. OF HOTEL MANAGEMENT, CATERING TECH. & APPLIED NUTR., BOMBAY-400 028, INDIA, Bd. 12, Nr. 4, Seiten 170-172,
- BELITZ; GROSCH: "Food Chemistry" 1999 , SPRINGER , BERLIN XP002263917 * Seite 488-489, Spalte 1, Kapitel 10.1.3.3- 10.1.3.4
- DEUTSCHE FORSCHUNGSANSTALT FÜR LEBENSMITTELCHEMIE: "Der kleine "Souci-Fachmann-Kraut"" 1991 , WISSENSCHAFTLICHE VERLAGSGESELLSCHAFT MBH , STUTTGART XP002263918 * Seite 24 *

## Beschreibung

Die vorliegende Erfindung betrifft ein Verfahren zur Herstellung und die Verwendung einer Enteralnahrung, insbesondere einer' Enterallösung oder - suspension.

Eine Enteralnahrung ist eine Nahrung, die entweder oral oder gastrointestinal dem Patienten oder Konsumenten zugeführt wird, ohne dass im Mund- und Rachenraum des Nutzers ein Nahrungsaufschluss stattgefunden hat. Enteralnahrungen liegen aus diesem Grund in der Regel in Form von Lösungen oder auch Suspensionen vor und finden sowohl bei Menschen als auch bei Tieren Verwendung. Üblicherweise enthalten Enteralvollnahrungen Fett-, Kohlenhydrat- und Eiweißkomponenten sowie häufig Zusatzstoffe, zum Beispiel zur Erhöhung ihrer Stabilität oder zur Geschmacksverbesserung. Ihre Herstellung umfasst in der Regel Pasteurisier-, Homogenisier- und Sterilisierungsschritte unter Einsatz höherer Temperaturen und Drücke.

Aus der US 4,497,800 ist eine Enteralvollnahrung bekannt. Die dort beschriebene Enterallösung weist einen niedrigen pH-Wert auf und ist demgemäß mikrobiell recht stabil. Als nachteilig erweist sich jedoch eine recht hohe Osmolalität und die Notwendigkeit Emulgatoren hinzu geben zu müssen.

Aus der EP 0 126 666 A ist eine weitere Enteralnahrung bekannt, die sich allerdings durch einen bitteren Geschmack auszeichnet.

Aus der US 4,959,350 ist eine flüssige Enteralnahrung mit ebenfalls geringem pH-Wert bekannt, die sich durch einen verbesserten Geschmack auszeichnet. Zur Erzielung mikrobieller Stabilität wurde die Lösung bei 85°C für 4 Sekunden pasteurisiert.

Den vorgenannten Enteralnahrungen ist gemeinsam, dass diese aus ernährungsphysiologischer Sicht verbesserungsfähig sind. So enthalten sie in der Regel solche glykämischen Kohlenhydrate, die zu einem schnellen und hohen Blutglucosespiegel führen und einen hohen, den Stoffwechsel belastenden Insulinbedarf aufweisen. Alternative Kohlenhydrate wie Fructose liefern hingegen keine ernährungsphysiologisch wertvolle Glucose und zersetzen sich während der Herstellung der enteralen Lösungen. Hinzu kommt, dass die bekannten Verfahren zur Herstellung von Enterallösungen aufgrund der zur Pasteurisierung und Sterilisierung eingesetzten Prozessbedingungen häufig einen Abbau von in der Enteralnahrung befindlichen Komponenten, insbesondere von Ketosen bewirken. In der Folge nimmt der Patient einerseits zu wenig der betreffenden, erwünschten Substanz und andererseits zuviel an Umwandlungsprodukten, zum Beispiel Produkten der Maillard-Reaktion wie gesundheitsschädigender AGEs (Advanced Glycation Endproducts), auf.

Der vorliegenden Erfindung liegt daher das technische Problem zugrunde, ein Verfahren zur Herstellung einer Enteralnahrung, insbesondere Ketosehaltiger Enterallösung oder -suspension, bereitzustellen, das die vorgenannten Nachteile überwindet, insbesondere zur technisch einfachen und kostengünstigen Bereitstellung einer ernährungsphysiologisch besonders wertvollen niedrig glykämischen und dennoch Glucose liefernden vorteilhaften, keimfreien oder keimreduzierten Enteralnahrung führt.

Die vorliegende Erfindung überwindet das ihr zugrundeliegende technische Problem durch die Bereitstellung eines Verfahrens zur Herstellung einer Isomaltulose-haltigen Enteralnahrung, insbesondere Enterallösung oder Enteralsuspension, umfassend die Schritte (a) Bereitstellen der Ausgangskomponenten Wasser, Fett, mindestens einer Stickstoff-haltigen Komponente und mindestens eines Kohlenhydrats, insbesondere Isomaltulose, (b) das anschließende Homogenisieren der bereitgestellten Ausgangskomponenten und (c) das anschließende Pasteurisieren der Ausgangskomponenten für 10 bis 30 Sekunden bei Temperaturen ≥ 135°C, vorzugsweise 135°C bis 137°C. Selbstverständlich kann die Reihenfolge der Schritte (b) und (c) vertauscht werden, das heißt das Verfahren betrifft in einer solchen Ausgestaltung ein Verfahren mit der Abfolge der Schritte (a) Bereitstellen der Ausgangskomponenten, (c) Pasteurisieren der bereitgestellten Ausgangskomponenten unter den genannten Bedingungen und (b) das anschlie-ßende Homogenisieren der pasteurisierten Ausgangskomponenten.

Die vorliegende Erfindung überwindet das ihr zugrundeliegende technische Problem auch durch die Bereitstellung eines Verfahrens zur Herstellung einer Isomaltulose-haltigen Enteralnahrung, insbesondere Enterallösung oder Enteralsuspension, umfassend die Schritte (a') Bereitstellen der Ausgangskomponenten Wasser, Fett, mindestens einer Stickstoff-haltigen Komponente und mindestens eines Kohlenhydrats, insbesondere Isomaltulose, (b') das anschließende Homogenisieren der bereitgestellten Ausgangskomponenten und (c') das anschließende Sterilisieren, insbesondere Autoklavieren der Ausgangskomponenten für 5 bis 15 Minuten bei Temperaturen ≥ 120°C, vorzugsweise 125°C bis 128°C. Selbstverständlich kann die Reihenfolge der Schritte (b') und (c') vertauscht werden, das heißt das Verfahren betrifft in einer solchen Ausgestaltung ein Verfahren mit der Abfolge der Schritte (a') Bereitstellen der Ausgangskomponenten, (c') Sterilisieren der bereitgestellten Ausgangskomponenten unter den genannten Bedingungen und (b') das anschließende Homogenisieren der autoklavierten Ausgangskomponenten.

Die Erfindung betrifft in einer weiteren Ausgestaltung ein Verfahren mit den vorgenannten Schritten (a), (b) und (c) oder (a), (c) oder (b), wobei im Anschluss an den letzten Verfahrensschritt des vorgenannten Verfahrens, gegebenenfalls nach Zugabe von Zusatzstoffen, eine Sterilisierung, insbesondere Autoklavierung der homogenisierten und pasteurisierten Ausgangskomponenten durchgeführt wird, vorzugsweise ein Autoklavieren bei Temperaturen ≥ 120°C, vorzugsweise 125°C bis 128°C, für einen Zeitraum von 5 bis 15 Minuten.

In einer weiteren bevorzugten Ausführungsform ist vorgesehen, dass der vorgenannte Pasteurisierungsschritt und/oder der vorgenannte Sterilisierungsschritt bei einem pH-Wert von 6,5 bis 8,0 vorzugsweise 6,5 bis 7,5 durchgeführt wird. In einer bevorzugten Ausführungsform kann vorgesehen sein, dass die Einstellung des pH-Wertes zu Beginn oder während der vorgenannten Herstellverfahren erfolgt.

Im Zusammenhang mit der vorliegenden Erfindung wird unter einer Enteralnahrung insbesondere eine keimreduzierte, im Wesentlichen keimfreie oder keimarme Enterallösung oder Enteralsuspension verstanden, die für die perorale oder gastrointestinale (Sondennahrung) Ernährung des menschlichen oder tierischen Körpers geeignet ist. Keime sind mikrobielle Organismen oder Vermehrungsprodukte solcher oder anderer Organismen, insbesondere Pilze, Sporen, Hefen, Bakterien, Bazillen, Protozoen, Algen, Flechten, Cyanobakterien etc. Im Zusammenhang mit der vorliegenden Erfindung wird unter Pasteurisieren ein hitzeverursachtes Abtöten von speziellen Keimarten und Viren verstanden, wobei vollständige Keim- und Virenfreiheit nicht erreicht wird. Unter Sterilisieren, insbesondere Autoklavieren das heißt Sterilisieren in einem Dampfdruckgefäß, wird ein auf das vollständige Abtöten von Keimen und Viren gerichtetes Verfahren verstanden, welches sich erfindungsgemäß insbesondere eines Erhitzens auf mindestens 120°C bedient.

Die Erfindung sieht also die Bereitstellung einer Enteralnahrung vor, die neben den, zum Beispiel für eine Vollernährung notwendigen, Nahrungsmittelkomponenten Wasser, Fett und Stickstoff-haltige Komponente als Kohlenhydrat Isomaltulose (auch als Palatinose bezeichnet) enthält. Isomaltulose liefert ernährungsphysiologisch günstige Glucose unter langsamer Freisetzung, ohne durch hohen Insulinbedarf den Stoffwechsel zu belasten. Die Isomaltulose erweist sich für die erfindungsgemäß hergestellte und eingesetzte Enteralnahrung also aufgrund ihrer langsamen Glucosefreisetzung und ihrer Insulinunabhängigen Verstoffwechselung bei vollem Energiewert als besonders vorteilhaft. Darüber hinaus zeichnet sich die erfindungsgemäße Herstellung und Verwendung durch einen reduzierten Gehalt an AGEs aus. In einer bevorzugten Ausführungsform der vorliegenden Erfindung liegt, neben der Isomaltulose kein weiteres Kohlenhydrat, insbesondere kein weiterer Zucker, in der Enteralnahrung vor. Isomaltulose ist in dieser Ausführungsform das einzige, alleinige Kohlenhydrat, insbesondere der einzige Zucker, in der Enteralnahrung. In einer weiteren bevorzugten Ausführungsform kann jedoch auch vorgesehen sein, dass Isomaltulose zusammen mit anderen Kohlenhydraten, zum Beispiel Glucose, Fructose, Invertzucker, Lactose, Maltose, Trehalulose, Maltodextrine, Pektin, Saccharose, Stärke, hydrolysierte Stärke, oder Zuckeraustauschstoffen wie Isomalt oder anderen Zuckeralkoholen, wie Lycasin, Mannit, Sorbit, Xylit, Erythrit, Maltit, Lactit, 1,6-GPS (6-O-α-D-Glucopyranosyl-D-sorbit), 1,1-GPM (1-O-α-D-Glucopyranosyl-D-mannit) oder 1,1-GPS (1-O-α-D-Glucopyranosyl-D-sorbit) etc. vorliegt. In letztgenannter Ausführungsform ist erfindungsgemäß besonders bevorzugt vorgesehen, dass die Isomaltulose einen Teil der üblicherweise in einer kommerziell erhältlichen Enteralnahrung vorhandenen Kohlenhydrate, insbesondere ≥30, ≥40, ≥50, ≥60, ≥70, ≥80, ≥90 oder ≥95 Gew.-% (bezogen auf Trockensubstanz aller Kohlenhydrate in der Enteralnahrung) ersetzt.

Die Erfindung sieht insbesondere vor, als Kohlenhydrat allein Isomaltulose oder in wesentlichen Anteilen in der Enteralnahrung einzusetzen und die Isomaltulose-haltigen Ausgangskomponenten für 10 bis 30 Sekunden bei Temperaturen von mindestens 13,5°C, insbesondere 135°C bis 137°C, zu pasteurisieren und/oder die Isomaltulose-haltigen Ausgangskomponenten für 5 bis 15 Min. bei Temperaturen von mindestens 120°C, insbesondere 125°C bis 128°C zu sterilisieren. Üblicherweise kann eine Reduktion von Kohlenhydratabbau durch niedrigere Temperaturen erreicht werden. Überraschenderweise hat sich gezeigt, dass auch bei hohen Temperaturen bei Verringerung der Verweilzeit eine Reduktion des Ketoseabbaues erzielt werden kann. Durch das Einhalten dieser Rezeptur und Pasteurisierungsbedingungen wird überraschenderweise ein besonders hoher Isomaltulosegehalt in der gebrauchsfertigen homogenisierten Enteralnahrung erhalten. Die auf diese Art und Weise schonend, gleichwohl aber keimfrei oder keimreduziert, erhaltene Enteralnahrung zeichnet sich in besonders vorteilhafter Weise durch eine hohe Lagerstabilität, eine hohe mikrobielle Stabilität und gute organoleptische Eigenschaften aus und weist einen angenehmen süßen Geschmack auf. Darüber hinaus wird Isomaltulose von den Glucosidasen der menschlichen Dünndarmwand lediglich verzögert gespalten. Dies resultiert verglichen zu schnell verdaulichen Kohlenhydraten in einem langsamen Anstieg der Blutglucose. Gleichzeitig wird auch die freigesetzte Fructose resorbiert. Beides zusammen führt dazu, dass Isomaltulose im Unterschied zu schnell verdaulichen, hochglykämischen Lebensmitteln kaum Insulin zur Verstoffwechselung benötigt. Weiterhin eignet sich Isomaltulose aufgrund des verzögerten Abbaus im Dünndarm besonders, um den oxidativen Metabolismus aufrechtzuerhalten. Die vorliegende Enteralnahrung eignet sich also hervorragend als "slow-release"-Nahrung, also Nahrung mit verzögerter, kontinuierlicher Kohlenhydratfreisetzung, die gleichzeitig aufgrund des geringeren Insulinbedarfs gerade für Personen, die an Störungen des Blutglucose-Stoffwechsels leiden, geeignet ist.

In einer weiteren bevorzugten Ausführungsform der Erfindung wird, wie erläutert, im Anschluss an den zeitlich letzten Schritt des erfindungsgemäßen Herstellungsverfahrens, das heißt nach dem Pasteurisier- oder Homogenisier-Schritt (b) oder (c) ein Sterilisierungsschritt der homogenisierten Ausgangskomponenten durchgeführt. Sofern die erhaltene Enterallösung nach dem Pasteurisieren oder Homogenisieren in sterile Gefäße abgefüllt wird, kann auf diesen Sterilisierschritt verzichtet werden.

Erfindungsgemäß kann auch vorgesehen sein, das Produkt nach der Pasteurisierung, Homogenisierung oder dem Sterilisieren, insbesondere Autoklavieren, zu trocknen, insbesondere zu sprühtrocknen und gegebenenfalls zu agglomerieren. Vor dem Gebrauch wird das erhaltene Pulver durch Auflösen in Wasser rekonstituiert.

Die Erfindung betrifft daher auch die mittels der in der vorliegenden technischen Lehre beschriebenen Verfahren hergestellten Isomaltulose-haltigen Enteralnahrungen.

In einer bevorzugten Ausführungsform der Erfindung betrifft diese eine Enteralnahrung mit 70 bis 80 Gew.-% (bezogen auf das Gesamtgewicht der Gesamtlösung oder -suspension) Wasser.

In einer weiteren bevorzugten Ausführungsform der Erfindung betrifft diese eine Enteralnahrung mit 1 bis 3,5 Gew.-% Stickstoff-haltiger Komponente (bezogen auf das Gesamtgewicht der Enteralnahrung).

In einer weiteren bevorzugten Ausführungsform der vorliegenden Erfindung betrifft diese eine Enteralnahrung mit 2 bis 4,5 Gew.-% Fett (bezogen auf das Gesamtgewicht der Enteralnahrung).

In einer weiteren bevorzugten Ausführungsform der vorliegenden Erfindung betrifft diese eine Enteralnahrung mit 6 bis 11 Gew.-% Kohlenhydratkomponente (bezogen auf das Gesamtgewicht der Enteralnahrung). In bevorzugter Ausführung beträgt der Isomaltulosegehalt von 1 bis 20 Gew.-%, vorzugsweise 5 bis 15 Gew.-% (bezogen auf das Gesamtgewicht der Lösung oder Suspension).

In einer weiteren bevorzugten Ausführungsform der vorliegenden Erfindung weist die Enteralnahrung, insbesondere die Enterallösung, einen pH-Wert von 2 bis 10, insbesondere 2 bis 8, vorzugsweise 6,5 bis 8,0, bevorzugt 6,5 bis 7,5 auf.

In einer weiteren bevorzugten Ausführungsform beträgt (in Bezug auf den Gesamtenergiegehalt) der Fettgehalt, insbesondere Triglyceride, 3 bis 60 %, der Gehalt an Stickstoff-haltiger Komponente 10 bis 35 % und der Gehalt an Kohlenhydraten 5 bis 87 %.

In einer besonders bevorzugten Ausführungsform liegt die Osmolalität gleich oder unter 350 Milliosmal.

In einer besonders bevorzugten Ausführungsform der vorliegenden Erfindung wird als Fett ein pflanzliches Fett, insbesondere ein pflanzliches Öl, zum Beispiel Maisöl, Kokosöl, Sojaöl oder Sonnenblumenöl oder Mischungen davon verwendet. Selbstverständlich ist es auch möglich, andere Fettkomponenten, insbesondere synthetische Öle zu verwenden.

In einer weiteren bevorzugten Ausführungsform werden als Stickstoff-haltige Komponente Proteine, Peptide, Aminosäuren, Gemische davon, Protein- oder Peptidhydrolysate, insbesondere hydrolysiertes Lactalbumin, hydrolysierte Molke, saure Molke, Käsemolke, Casein, hydrolysiertes Casein, Caseinate, hydrolysiertes Sojabohnenprotein oder/und freie Aminosäuren verwendet. In bevorzugter Ausführungsform werden Stickstoff-haltige Komponenten verwendet, die Proteine pflanzlicher Herkunft darstellen oder davon hergestellt werden. Erfindungsgemäß können beispielsweise Proteinhydrolysate von Raps, Bohne, Weizen, Sesam oder Erbse verwendet werden. Selbstverständlich können auch Mischungen solcher Hydrolysate eingesetzt werden.

In einer weiteren bevorzugten Ausführungsform ist vorgesehen, dass die Ausgangskomponenten des Schrittes (a) auch Geschmacksstoffe, Puffer, Salze, Konservierungsstoffe, Geruchsstoffe, weitere Süßungsmittel, Mineralien, Vitamine, Ballaststoffe, nahrungsmittelverträgliche Säuren, Spurenelemente, Elektrolyte und/oder Emulgatoren, pharmazeutisch wirksame Substanzen, Antibiotika, Antioxidantien etc. umfassen.

Die Erfindung betrifft auch die Verwendung von Isomaltulose in Enteralnahrungen oder zur Herstellung von Enteralnahrungen, hergestellt nach einem der vorhergehenden Verfahren als niedrigglykämisches Kohlenhydrat, das heißt mit niedrigem Insulinbedarf, wobei die Enteralnahrung für gesunde menschliche oder tierische Körper oder für menschliche oder tierische Körper mit gestörtem Glucose- und/oder Insulinstoffwechsel geeignet ist.

Weitere vorteilhafte Ausgestaltungen der vorliegenden Erfindung ergeben sich aus den Unteransprüchen.

Die Erfindung wird anhand der folgenden Beispiele näher erläutert.

### Beispiel 1:

### Herstellung und Pasteurisierung einer Enterallösung mit Palatinosezusatz (durch UHT-Erhitzung (UHT: Ultra Hochtemperatur)).

A) Die Lösungskomponenten gemäß nachstehender Rezeptur (Abschnitt B)) werden in einem Becherglas in der Reihenfolge Salze, Vitamine, Kohlenhydrate und abschließend Proteine in einer Vorlage in Wasser aufgenommen und mittels eines Ultra-Turrax-Rührwerkes homogenisiert. Die homogenisierte Masse wird dann mittels einer Pumpe durch die Versuchsanlage gefördert. Die Versuchsanlage ist aus den Abteilungen Zulauf, Vorwärmer, UHT-Erhitzer, Heißhalter, Kühler und Auslauf zusammengesetzt. Es handelt sich um eine indirekte, mit Dampf beheizte UHT-Anlage, die üblicherweise für die UHT-Erhitzung von Milch eingesetzt wird. Die Verweilzeit im Heißhaltesystem wird durch die Pumpenförderleistung variinert. Mittels der UHT-Anlage wird die erfindungsgemäße Pasteurisierung gemäß der aus der folgenden Tabelle 1 hervorgehenden Versuchs-Zeiten und -Temperaturen durchgeführt.

Der analytische Nachweis der Kohlenhydratbestandteile erfolgt mittels High Performance Anion Exchange Chromatography (HPAEC) mit NaOH als Eluent und amperometrischer Detektion.

**Tabelle 1 Ergebnisse:**

| | Ansatz 1 | Ansatz 2 | Ansatz 3 |
|---|---|---|---|
| **T (Temperatur) [°C]** | 130 | 135 | 140 |
| **t (Zeit) [sek]** | 50 | 30 | 10 |
| | (Referenz) | | |
| Isomaltulose [g/kg] | | | |
| Lösung vor dem UHT-Schritt | 101,07 | 100,23 | 100,07 |
| Lösung nach dem UHT-Schritt | 69,25 | 72,52 | 81,20 |
| Isomaltuloseabbau [%] | 31 | 28 | 19 |

Die erhaltenen Keim- und Virenzahlen waren in allen drei Ansätzen im Wesentlichen identisch. Der Isomaltuloseabbau war jedoch bei dem erfindungsgemäßen Vorgehen (Ansätze 2 und 3) deutlich gegenüber einem Kontrollansatz mit reduzierter Temperatur und längerer Pasteurisierzeit reduziert. Durch die Verkürzung der Inkubationszeiten gelang eine Abbaureduzierung um cirka ein Drittel.

B) Beispiel für eine Rezeptur mit Isomaltulose

| **Rohstoff** | **Kg/100kg** |
|---|---|
| Wasser , | 76, 379001 |
| Isbmaltulose | 10,000000 |
| Glucidex 12 Maltodextrin 10 DE | 5,295000 |
| Calciumcaseinat, sprühgetrocknet | 3,400000 |
| Fettmischurig, Standard | 3,110000 |
| Natriumcaseinat, sprühgetrocknet | 0,900000 |
| Kaliumchlorid | 0,185500 |
| Emulgator Myverol 18-0, des. Monoglycerid | 0,125000 |
| Tri-Kaliumcitrat, 1-Hydrat | 0,110000 |
| Kaliumdihydrogenphosphat, K11-01 | 0,105000 |
| Emulgator Halocithin 02-F | 0,080000 |
| Tri-Natriumcitrat Dihydrat, Grad 6090 | 0,080000 |
| Tri-Calciumplosphat | 0,060000 |
| Glucidex 21 Maltodextrin 20 DE | 0,044122 |
| Magnesiumoxid, schwer | 0,040000 |
| Kaliumdihydrocitrat Anhydrat | 0,030000 |
| Cholin-bitartrat, beschichtet | 0,022000 |
| Vitamin C, pulverisiert | 0,013600 |
| Eisen-II-Lactat | 0,005000 |
| Zinksulfat-1-Hydrat | 0,002750 |
| Natriumchlorid | 0,002000 |
| Nicotinamid | 0,002000 |
| Antioxidanz Ascorbylpalmitat | 0,001500 |
| Vit. A-Acetat 325 | 0,001400 |
| Kaliumjodid, 1% I Verreibung | 0,001150 |
| Cu-II-Gluconat | 0,000845 |
| Mn-II-Sulfat-1-Hydrat | 0,000715 |
| Ca-D-Pantothenat | 0,000550 |
| Natriummolybdat 1% Molybdän Verreibung | 0,000500 |
| Vitamin D3 | 0,000450 |
| Natriumfluorid | 0,000400 |
| Natriumselenit 1% Seien Verreibung | 0,000300 |
| Vitamin B12 0,1% | 0,000240 |
| Chrom-III-Chlorid 1% Chrom Verreibung | 0,000225 |
| Vitamin B6-HCl | 0,000225 |
| Vitamin B2 | 0,000187 |
| Vitamin Bl-HCl | 0,000150 |
| Vitamin K1 5% SD | 0,000060 |
| Folsäure | 0,000024 |
| Biotin, d | 0,000006 |
| Summe | 100,000000 |

### Beispiel 2:

### Sterilisation durch Autoklavieren

Die Lösungskomponenten gemäß der Rezeptur aus Beispiel 1 (Abschnitt B) werden in einem Becherglas in der Reihenfolge Salze, Vitamine, Kohlenhydrate und abschließend Protein in einer Vorlage in Wasser aufgenommen und mittels eine Ultra-Turrax-Rührwerkes homogenisiert. Die homogenisierte Masse wird dann in ein Autoklaviergefäß überführt und in einem Dampf-Laborautoklaven sterilisiert. Gemäß dieser Beschreibung wird die erfindungsgemäße Autoklavierung (Sterilisation) mit den aus folgender Tabelle 2 hervorgehenden Versuchs-Zeiten und -Temperaturen durchgeführt.

Der analytische Nachweis der Kohlenhydratbestandteile erfolgt mittels High Preformance Anion Exchange Chromatography (HPAEC) mit NaOH als Eluent und amperometrischer Detektion.

**Tabelle 2 Ergebnisse:**

| | | Ansatz 1 | Ansatz 2 | Ansatz 3 |
|---|---|---|---|---|
| **T (Temperatur) [°C]** | | 115 | 121 | 128 |
| **t (Zeit) [min]** | | 30 | 15 | 5 |
| **p (bar_{abs.})** | | 1,7 | 2,1 | 2, 5 |
| | | (Referenz) | | |
| Isomaltulose [g/kg] | | | | |
| | Lösung vor dem Autoklavieren | 100,25 | 100,13 | 100,26 |
| | Lösung nach dem Autoklavieren | 58,71 | 61,27 | 69,37 |
| Isomaltuloseabbau [%] | | 41 | 39 | 31 |

Die erhaltenen Keim- und Virenzahlen waren in allen drei Ansätzen im Wesentlichen identisch. Der Isomaltuloseabbau war jedoch beim erfindungsgemäßen Vorgehen (Ansätze 2 und 3) deutlich gegenüber einem Kontrollansatz mit reduzierter Temperatur und längerer Sterilisationszeit reduziert. Durch die Verkürzung der Inkubationszeiten gelang eine Abbaureduzierung um zirka 33 %.

### Beispiel 3:

### Herstellung, Pasteurisierung (also UHT-Erhitzung) und Sterilisierung (Autoklavieren) einer Enterallösung mit Palatinosezusatz.

Die Lösungskomponenten gemäß der Rezeptur aus Beispiel 1 (Abschnitt B) werden in einem Becherglas in der Reihenfolge Salze, Vitamine, Kohlenhydrate und abschließend Proteine in einer Vorlage in Wasser aufgenommen und mittels eines Ultra-Turrax-Rührwerkes homogenisiert. Die homogenisierte Masse wird dann mittels einer Pumpe durch die Versuchsanlage gefördert. Die Versuchsanlage ist aus den Abteilungen Zulauf, Vorwärmer, UHT-Erhitzer, Heißhalter, Kühler und Auslauf zusammengesetzt. Es handelt sich um eine indirekte, mit Dampf beheizte UHT-Anlage, die üblicherweise für die UHT-Erhitzung von Milch eingesetzt wird. Die Verweilzeit im Heißhaltesystem wird durch die Pumpenförderleistung variiert. Mittels der UHT-Anlage wird die erfindungsgemäße Pasteurisierung gemäß der aus der folgenden Tabelle 3 hervorgehenden Versuchs-Zeiten und - Temperaturen durchgeführt.

Der analytische Nachweis der Kohlenhydratbestandteile erfolgt mittels High Performance Anion Exchange Chromatography (HPAEC) mit NaOH als Eluent und amperometrischer Detektion.

**Tabelle 3 Ergebnisse:**

| | Ansatz 1 | Ansatz 2 | Ansatz 3 |
|---|---|---|---|
| T (Temperatur) [°C] | 130 | 135 | 140 |
| t (Zeit) [sek] | 50 | 30 | 10 |
| | (Referenz) | | |
| Isomaltulose [g/kg] | | | |
| Lösung vor dem UHT-Schritt | 100,65 | 99,38 | 100,05 |
| Lösung nach dem UHT-Schritt | 66,38 | 76,36 | 83,72 |
| Isomaltuloseabbau (%) | 34 | 23 | 16 |

Das Produkt von Reaktionsansatz 3 (d.h. das Produkt mit dem höchsten Rest-Isomaltuloseanteil nach dem Pasteurisierungsschritt) wird in ein Autoklaviergefäß überführt und in einem Dampf-Laborautoklaven sterilisiert. Gemäß dieser Beschreibung wird die erfindungsgemäße Autoklavierung (Sterilisation) mit den aus folgender Tabelle 4 hervorgehenden Versuchs-Zeiten und -Temperaturen durchgeführt.

Der analytische Nachweis der Kohlenhydratbestandteile erfolgt mittels High Performance Anion Exchange Chromatography (HPAEC) mit NAOH als Eluent und amperometrischer Detektion.

Die enthaltenen Keim- und Virenzahlen waren in allen drei Ansätzen im Wesentlichen identisch. Der Isomaltuloseabbau war jedoch beim erfindungsgemäßen Vorgehen (Ansätze 2 und 3, 5 und 6) deutlich gegenüber den Kontrollansätzen mit reduzierter Temperatur und längerer Sterilisationszeit reduziert. Durch die Verkürzung der Inkubationszeiten gelang eine Abbaureduktion um ca. 40% für den Autoklavierschritt und von 22% für das Gesamtherstellverfahren.

**Tabelle 4 Ergebnisse:**

| | | Ansatz 4 | Ansatz 5 | Ansatz 6 |
|---|---|---|---|---|
| T (Temperatur) [°C] | | 115 | 121 | 128 |
| t (Zeit) [min] | | 30 | 15 | 5 |
| P (bar_{abs}.) | | 1,7 | 2,1 | 2,5 |
| | | (Referenz) | | |
| Isomaltulose [g/kg] | | | | |
| | Lösung vor dem Autoklavieren | 83,72 | 83,72 | 83,72 |
| | Lösung nach dem Autoklavieren | 60,28 | 65,30 | 70,30 |
| Isomaltuloseabbau beim Autoklavieren [%] beim Auto- | | 28 28 | 22 22 | 16 16 |
| Isomaltuloseabbau qesamt [%] | | 40 | 35 | 31 |

## Patentansprüche

1. Verfahren zur Herstellung einer Isomaltulose-haltigen Enteralnahrung umfassend die Schritte:
- Bereitstellen (a, a') der Ausgangskomponenten Wasser, Fett, mindestens einer Stickstoff-haltigen Komponente und Kohlenhydrate unter Einschluss von Isomaltulose;
- Abtöten von Keimen und Viren der Ausgangskomponenten durch:
- Pasteurisieren (c), für 10 bis 30 Sekunden bei ≥ 135°C, oder
- Autoklavieren (c'), für 5 bis 15 min. bei ≥ 120°C;
und
- Homogenisieren (b, b') der Ausgangskomponenten vor oder nach dem Verfahrensschritt des Abtötens von Keimen und Viren (c, c').

2. Verfahren nach Anspruch 1, wobei im Anschluss an die Verfahrensschritte Homogenisieren (b) und Pasteurisieren (c) eine Sterilisierung der homogenisierten und pasteurisierten Ausgangskomponenten durchgeführt wird, vorzugsweise ein Autoklavieren bei ≥ 120°C, für 5 bis 15 min.

3. Verfahren nach Anspruch 1 oder 2, wobei die Pasteurisierungstemperatur bei 135°C bis 137°C liegt.

4. Verfahren nach Anspruch 1 oder 2, wobei das Autoklavieren bei 125°C bis 128°C durchgeführt wird.

5. Verfahren nach einem der vorhergehenden Ansprüche, wobei die Pasteurisierung und/oder das Autoklavieren bei einem pH-Wert von 6,5 bis 8,0, vorzugsweise 6,5 bis 7,5 durchgeführt wird.

6. Verfahren nach einem der vorhergehenden Ansprüche, wobei die Nahrung in flüssiger Form, insbesondere in Form einer Lösung oder Suspension vorliegt.

7. Verfahren nach einem der vorhergehenden Ansprüche, wobei die Stickstoff-haltige Komponente mindestens ein Protein, mindestens ein Peptid, mindestens eine Aminosäure, ein Gemisch von Aminosäuren oder ein Protein- oder Peptid-Hydrolysat oder ein Gemisch von mindestens zwei der vorgenannten Komponenten ist.

8. Verfahren nach einem der vorhergehenden Ansprüche, wobei die Stickstoff-haltige Komponente Sojabohnenproteinhydrolysat, Caseinat, hydrolysiertes Casein, hydrolysiertes Molkenprotein, hydrolysiertes Lactalbumin, oder ein Gemisch davon ist.

9. Verfahren nach einem der vorhergehenden Ansprüche, wobei das Fett in Form von pflanzlichem Fett, insbesondere pflanzlichen Ölen, vorliegt.

10. Verfahren nach einem der vorhergehenden Ansprüche, wobei das pflanzliche Öl Maisöl, Kokosöl, Sonnenblumenöl, Sojaöl oder ein Gemisch davon ist.

11. Verfahren nach einem der vorhergehenden Ansprüche, wobei neben Isomaltulose als Kohlenhydrat Maltodextrine, Saccharose, Glucose, Fructose, Trehalulose, Invertzucker, Lactose, Lactit, Maltit, Erythrit, Xylit, Mannit, Sorbit, Lycasin, Isomalt, Maltose, Pektin, Stärke, hydrolysierte Stärke oder ein anderer Zuckeralkohol oder Zuckeralkoholgemisch oder ein Gemisch davon eingesetzt wird.

12. Verfahren nach einem der Ansprüche 1 bis 10, wobei die Isomaltulose das einzige Kohlenhydrat in der Enteralnahrung ist.

13. Verwendung von Isomaltulose in Enteralnahrung für den gesunden menschlichen oder tierischen Körper, hergestellt nach dem Verfahren nach einem der Ansprüche 1 bis 12, als niedrig glykämisches Kohlenhydrat.

14. Verwendung nach Anspruch 13, wobei der menschliche oder tierische Körper einen gestörten Glucose- und/oder Insulinstoffwechsel hat.

## Claims

1. Process for manufacturing an isomaltulose-containing enteral food comprising the steps of:
- providing (a, a') the starting components water, fat, at least one nitrogen-containing component and carbohydrates with the inclusion of isomaltulose;
- destroying germs and viruses in the starting components by means of:
- pasteurising (c) for 10 to 30 seconds at ≥ 135 °C, or
- autoclaving (c') for 5 to 15 min at ≥ 120 °C;
and
- homogenising (b, b') the starting components before or after the step of destroying the germs and viruses (c, c').

2. Process according to claim 1, wherein a sterilisation, preferably autoclaving at ≥ 120 °C for 5 to 15 min, of the homogenised and pasteurised starting components is effected following the steps of homogenising (b) and pasteurising (c).

3. Process according to claim 1 or 2, wherein the pasteurising temperature is 135 °C to 137 °C.

4. Process according to claim 1 or 2, wherein the autoclaving is effected at 125 °C to 128 °C.

5. Process according to one of the preceding claims, wherein the pasteurisation and/or the autoclaving is effected at a pH value of 6.5 to 8.0, preferably 6.5 to 7.5.

6. Process according to one of the preceding claims, wherein the food is in the form of a liquid, in particular in the form of a solution or suspension.

7. Process according to one of the preceding claims, wherein the nitrogen-containing component is at least one protein, at least one peptide, at least one amino acid, a mixture of amino acids, or a protein or peptide hydrolysate, or a mixture of at least two of said components.

8. Process according to one of the preceding claims, wherein the nitrogen-containing component is soy bean protein hydrolysate, caseinate, hydrolysed casein, hydrolysed whey protein, hydrolysed lactalbumin, or a mixture thereof.

9. Process according to one of the preceding claims, wherein the fat is present in the form of vegetable fat, in particular in the form of vegetable oils.

10. Process according to one of the preceding claims, wherein the vegetable oil is corn oil, coconut oil, sunflower oil, soy oil or a mixture thereof.

11. Process according to one of the preceding claims, wherein in addition to isomaltulose, maltodextrines, saccharose, glucose, fructose, trehalulose, invert sugar, lactose, lactitol, maltitol, erythritol, xylitol, mannitol, sorbitol, lycasin, isomalt, maltose, pectin, starch, hydrolysed starch or another sugar alcohol or a mixture of sugar alcohol, or a mixture thereof, is used as carbohydrate.

12. Process according to one of claims 1 to 10, wherein isomaltulose is the only carbohydrate in the enteral food.

13. Use of isomaltulose in enteral food for the healthy human or animal body, produced according to the process of one of claims 1 to 12, as a low glycemic carbohydrate.

14. Use according to claim 13, wherein the human or animal body has a disturbed glucose and/or insulin metabolism.

## Revendications

1. Procédé pour la préparation d'un aliment entéral contenant de l'isomaltulose, comprenant les étapes :
- fourniture (a, a') des composants de départ eau, graisse, d'au moins un composant azoté et de glucides en incluant de l'isomaltulose ;
- destruction des germes et virus des composants de départ par
- pasteurisation (c) pendant 10 à 30 secondes à ≥ 135°C ou
- passage dans un autoclave (c') pendant 5 à 15 minutes à ≥ 120°C;
et
- homogénéisation (b, b') des composants de départ avant ou après l'étape de procédé de la destruction des germes et virus (c, c').

2. Procédé selon la revendication 1, dans lequel une stérilisation des composants de départ homogénéisés et pasteurisés est effectuée à la suite des étapes de procédé de l'homogénéisation (b) et de la pasteurisation (c), de préférence un passage dans un autoclave à ≥ 120°C pendant 5 à 15 minutes.

3. Procédé selon la revendication 1 ou 2, dans lequel la température de pasteurisation est de 135°C à 137°C.

4. Procédé selon la revendication 1 ou 2, dans lequel le passage dans l'autoclave est effectué de 125°C à 128°C.

5. Procédé selon l'une des revendications précédentes, dans lequel la pasteurisation et/ou le passage dans l'autoclave est effectué à une valeur de pH de 6,5 à 8,0, de préférence de 6,5 à 7,5.

6. Procédé selon l'une des revendications précédentes, dans lequel l'aliment est sous forme liquide, en particulier sous la forme d'une solution ou d'une suspension.

7. Procédé selon l'une des revendications précédentes, dans lequel le composant azoté est au moins une protéine, au moins un peptide, au moins un acide aminé, un mélange d'acides aminés ou un hydrolysat de protéine ou de peptide ou un mélange d'au moins deux des composants précités.

8. Procédé selon l'une des revendications précédentes, dans lequel le composant azoté est un hydrolysat de protéine de soja, de la caséinate, de la caséine hydrolysée, de la protéine lactosérique hydrolysée, de la lactalbumine hydrolysée ou un mélange de ceux-ci.

9. Procédé selon l'une des revendications précédentes, dans lequel la graisse est présente sous la forme de graisse végétale, en particulier d'huiles végétales.

10. Procédé selon l'une des revendications précédentes, dans lequel l'huile végétale est de l'huile de maïs, de l'huile de coco, de l'huile de tournesol, de l'huile de soja ou un mélange de celles-ci.

11. Procédé selon l'une des revendications précédentes, dans lequel est utilisé comme glucide, outre de l'isomaltulose, de la maltodextrine, du saccharose, du glucose, du fructose, du tréhalulose, du sucre inverti, du lactose, du lactitol, du maltitol, de l'érythritol, du xylitol, du mannitol, du sorbitol, de la lycasine, de l'isomalt, du maltose, de la pectine, de l'amidon, de l'amidon hydrolysé, ou un autre alcool de sucre ou mélange d'alcools de sucre ou un mélange de ceux-ci.

12. Procédé selon l'une des revendications 1 à 10, dans lequel l'isomaltulose est le seul glucide dans l'aliment entéral.

13. Utilisation d'isomaltulose en tant que glucide à faible index glycémique dans l'aliment entéral pour le corps humain ou animal sain, préparé suivant le procédé selon l'une des revendications 1 à 12.

14. Utilisation selon la revendication 13, le corps humain ou animal ayant une assimilation de glucose et/ou d'insuline insuffisante.
